# EUROPÄISCHE PATENTANMELDUNG

(11) **EP 0 577 560 A1**
(43) Veröffentlichungstag der Anmeldung: **05.01.1994**
(21) Anmeldenummer: 93810454.4
(22) Anmeldetag: 24.06.1993
(51) Int. Cl.: C07D 513/06, H01B 1/12, C07C 331/30

(54) **Substituierte Naphtho[1,8-de:5,4-d'e']bis[1,3]thiazine, Verfahren zu deren Herstellung und deren elektrisch leitende charge-transfer komplexe**

(30) Priorität: 02.07.1992 CH 2087/92
(71) Anmelder: CIBA-GEIGY AG, CH-4002 Basel (CH)
(72) Erfinder: Zambounis, John, Dr., CH-3280 Murten (CH)

(57) **Zusammenfassung**

Verbindungen der Formel I 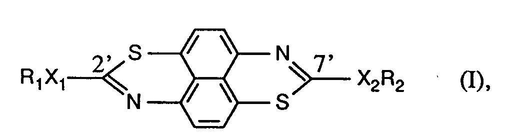
worin
X₁ und X₂ unabhängig voneinander O, S oder Se bedeuten,
und R₁ und R₂ unabhängig voneinander ein unsubstituierter oder mit NH₂, C₁-C₆-Alkyl, C₁-C₆-Alkoxy, Phenyl oder C₃-C₈-Cycloalkyl substituierter einwertiger Rest eines aliphatischen oder aromatischen Kohlenwasserstoffs mit 1 bis 20 C-Atomen darstellen.

Die Verbindungen der Formel I können elektrisch leitende Charge-Transfer Komplexe bilden.

## Beschreibung

Die vorliegende Erfindung betrifft Naphtho[1,8-de:5,4-d'e']bis[1,3]thiazine, die in den 2',7'-Stellungen mit organischen Thio-, Oxy- oder Selenoresten substituiert sind, ein Verfahren zu deren Herstellung und deren Verwendung, sowie 4,8-Dibrom-1,5-diisothiocyanatonaphthalin als Zwischenprodukt.

Hochleitende Charge-Transfer Komplexe von Pyren sind zum Beispiel von V. Enkelmann in J. de Phys., C3, 44, S.1147-1152 (1983) beschrieben. Diese Komplexe sind nicht lagerstabil und zersetzen sich nach kurzer Zeit. Mercaptopyrene und deren elektrisch leitende Charge-Transfer Komplexe mit anorganischen Anionen sind in der DE-A-3 814 534 beschrieben. In der DE-A-2 224 746 ist tetracyclisches Chlor-bis-1,3-thiazin als Zwischenprodukt zur Herstellung von Pflanzenschutzmitteln und Farbstoffen beschrieben.

Es wurde nun überraschend gefunden, dass mit einem neuen Verfahren in den 2',7'-Stellungen mit organischen Thio-, Oxy- oder Selenoresten substituierte Naphtho[1,8-de:5,4-d'e']bis[1,3]thiazine hergestellt werden können, die mit anorganischen Anionen elektrisch leitende Charge-Transfer Komplexe bilden. Die Charge-Transfer Komplexe kristallisieren in der Regel nadelförmig und weisen überraschend hohe elektrische Leitfähigkeiten auf.

Ein Gegenstand der Erfindung sind Verbindungen der Formel I 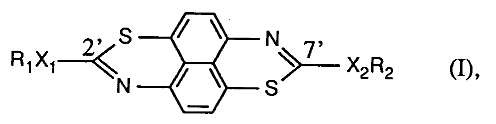
worin
X₁ und X₂ unabhängig voneinander O, S oder Se bedeuten,
und R₁ und R₂ unabhängig voneinander ein unsubstituierter oder mit NH₂, C₁-C₆-Alkyl, C₁-C₆-Alkoxy, Phenyl oder C₃-C₈-Cycloalkyl substituierter einwertiger Rest eines aliphatischen oder aromatischen Kohlenwasserstoffs mit 1 bis 20 C-Atomen darstellt. Bei R₁ und R₂ handelt es sich bevorzugt um gleiche Reste.

Die Kohlenwasserstoffreste enthalten bevorzugt 1 bis 18, besonders bevorzugt 1 bis 12 und insbesondere bevorzugt 1 bis 8 C-Atome.

Geeignete aliphatische Kohlenwasserstoffreste für R₁ und R₂ sind z.B. C₁-C₁₈-, bevorzugt C₁-C₁₂-, besonders bevorzugt C₁-C₆-Alkyl, C₃-C₈- oder bevorzugt C₄-C₆-Cycloalkyl, C₇-C₁₂-Aralkyl und besonders bevorzugt C₇-C₁₂-Phenylalkyl. Einige Beispiele sind Ethyl, Methyl, Propyl und lineares oder verzweigtes Dodecyl, Undecyl, Decyl, Nonyl, Octyl, Heptyl, Hexyl, Pentyl und Butyl. Einige Beispiele für Cycloalkyl sind Cyclopropyl, Cyclobutyl, Cyclopentyl, Cyclohexyl, Cycloheptyl und Cyclooctyl. Einige Beispiele für Aralkyl sind Benzyl und Phenylethyl.

Geeignete aromatische Kohlenwasserstoffreste für R₁ und R₂ sind z.B. C₆-C₁₈- und bevorzugt C₆-C₁₂-Aryl. Beispiele sind besonders Phenyl und Naphthyl.

In einer bevorzugten Ausführungsform sind R₁ und R₂ unsubstituiertes oder wie zuvor definiert substituiertes C₁-C₁₈-Alkyl besonders C₁-C₁₂-Alkyl, C₅- oder C₆-Cycloalkyl, Phenyl oder Benzyl.

X₁ und X₂ haben bevorzugt die gleiche Bedeutung. Bevorzugt sind X₁ und/oder X₂ O oder S; besonders bevorzugt sind X₁ und X₂ gleich und stehen für O oder S.

Beispiele für Cycloalkylsubstituenten sind Cyclopropyl, Cyclopentyl, Cyclohexyl, Cycloheptyl und Cyclooctyl. Bevorzugte Cycloalkylreste sind Cyclopentyl und Cyclohexyl.

Beispiele für Alkylsubstituenten, die bevorzugt 1-4 C-Atome enthalten, sind Methyl, Ethyl, n- und i-Propyl, und n-, i- und t-Butyl.

Beispiele für Alkoxysubstituenten für R₁ und R₂, die bevorzugt 1-4 C-Atome enthalten, sind Methoxy, Ethoxy, n-Propoxy und t-Butoxy.

Eine bevorzugte Untergruppe von Substituenten für R₁ und R₂ ist Methyl, Ethyl, Methoxy und Ethoxy.

Bevorzugte Beispiele für R₁ und R₂ sind Methyl, Ethyl, n-Propyl, n-Butyl, Benzyl und Phenyl.

Einige Beispiele für Verbindungen der Formel I sind 2',7'-Bis(methylthio)-naphtho-[1,8-de:5,4-d'e']bis[1,3]thiazin, 2',7'-Bis(methoxy)-naphtho[1,8-de:5,4-d'e']-bis[1,3]thiazin, 2'-Methylthio-7'-ethoxy-naphtho[1,8-de:5,4-d'e']bis[1,3]thiazin, und 2'-n-Butylthio-7'-methylthio-naphtho[1,8-de:5,4-d'e']bis[1,3]thiazin.

Die Verbindungen der Formel I können in einfacher Weise nach einem neuen Verfahren erhalten werden.

Ein anderer Gegenstand der Erfindung ist ein Verfahren zur Herstellung von Verbindungen der Formel I, das dadurch gekennzeichnet ist, dass man
i) 4,8-Dibrom-1,5-diaminonaphthalin der Formel II, gegebenenfalls als Salz, 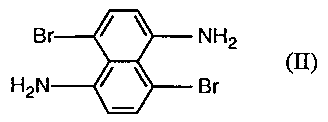 in Gegenwart eines inerten Lösungsmittels und Na₂CO₃ mit Thiophosgen zu 4,8-Dibrom-1,5-diisothiocyanatonaphthalin der Formel III 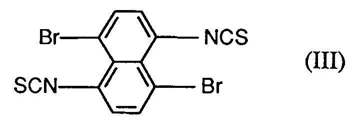 umsetzt,
ii) das 4,8-Dibrom-1,5-diisothiocyanatonaphthalin der Formel III in Gegenwart eines Lösungsmittels entweder durch Umsetzung mit 2 Äquivalenten eines einwertigen Metall- oder Ammoniumsalzes R₁X₁⁻ M⁺ oder eines Erdalkalimetallsalzes (R₁X₁⁻)₂E²⁺ in eine Verbindung der Formel Ia 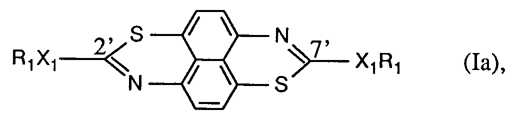 umwandelt, worin M⁺ ein einwertiges Metallkation oder ein Ammoniumkation R₃R₄R₅R₆N⁺ darstellt und R₃, R₄, R₅ und R₆ unabhängig voneinander H oder C₁-C₄-Alkyl und E²⁺ ein Erdalkalimetallkation bedeuten,
   oder stufenweise erst durch Umsetzung mit 1 Äquivalent eines einwertigen Metall- oder Ammoniumsalzes R₁X₁⁻M⁺ oder eines Erdalkalimetallsalzes (R₁X₁⁻)₂E²⁺ und dann mit 1 Äquivalent eines anderen einwertigen Metall- oder Ammoniumsalzes R₂X₂⁻M⁺ oder eines anderen Erdalkalimetallsalzes (R₂X₂⁻)₂E²⁺, in eine Verbindung der Formel I 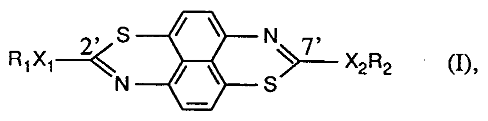 umwandelt, worin R₁, R₂, X₁ und X₂ die zuvor angegebenen Bedeutungen haben.

Beispiele für M⁺ sind Li⁺, Na⁺, K⁺, Cu⁺, Tl⁺, NH₄⁺, (CH₃)₃NH⁺ und (C₂H₅)₃NH⁺. Beispiele für E²⁺ sind Mg²⁺, Ca²⁺ und Sr²⁺. Cu⁺ ist zum Beispiel als C₆H₅SCu käuflich. Bevorzugt bedeutet M⁺ Na⁺, K⁺, NH₄⁺, (CH₃)₃NH⁺ und (C₂H₅)₃NH⁺. In einer anderen bevorzugten Ausführungsform bedeutet M⁺ eine Alkalimetallkation.

Die Stufen der Gesamtreaktion können bei einer Temperatur von 5 bis 80 °C, vorzugsweise 15 bis 60 °C durchgeführt werden.

Geeignete Lösungsmittel für die beiden Reaktionsstufen sind vorteilhaft polar und aprotisch, und schliessen z.B. folgende Lösungsmittel ein: Sulfone; Sulfoxide; N,N'-Tetrasubstituierte Harnstoffe; N-Alkylierte Lactame oder N-Dialkylierte Säureamide; Ether; gegebenenfalls halogenierte aliphatische, cycloaliphatische oder aromatische Kohlenwasserstoffe; Carbonsäureester und Lactone; Nitrile.

Beispiele sind:
Sulfone: Dimethylsulfon, Diethylsulfon.
Sulfoxide: Dimethylsulfoxid, Diethylsulfoxid.
N,N-Tetrasubstituierter Harnstoff: N-Methylethyl-N'-methylethylharnstoff, Tetramethylharnstoff.
N-Alkylierte Lactame: N-Methylpyrrolidon, N-Ethylpyrrolidon.
N-Dialkylierte Säureamide: N-Dimethylformamid, N-Dimethylacetamid.
Ether: Diethylenglykoldimethylether, Diethylenglykoldiethylether, Tetrahydrofuran. Aliphatische Kohlenwasserstoffe: Dichlormethan, Hexan, Chloroform, Trichlorethan, Tetrachlorethan.
Aromatische Kohlenwasserstoffe: Chlorbenzol, Dichlorbenzol.
Carbonsäureester: Essigsäuremethylester, Essigsäureethylester.
Nitrile: Benzonitril, Phenylacetonitril, Acetonitril.

Bevorzugte Lösungsmittel sind Dichlormethan, Tetrahydrofuran und Dimethylformamid.

Das erfindungsgemässe Verfahren, besonders die zweite Stufe, wird zweckmässig unter einer inerten Atmosphäre, z.B. Edelgasen wie Argon oder unter Stickstoff durchgeführt.

Das Produkt jeder Stufe kann in an sich bekannterweise isoliert werden, z.B. durch Dekantieren, Filtration oder Destillation. Anschliessend können die Produkte mittels üblichen Methoden wie zum Beispiel Kristallisation oder chromatographische Methoden gereinigt werden.

Die Herstellung des Ausgangsproduktes 4,8-Dibrom- 1,5-diaminonaphthalin der Formel II ist an sich bekannt und von J. S. Whitehurst in J. Chem. Soc., S.221-226 (1951) beschrieben. 4,8-Dibrom-1,5-ditoluol-p-sulfonamidonaphthalin wird in konzentrierter Säure gelöst, z.B. in H₂SO₄, und unter Lichtausschluss 24 h stehen gelassen. Die Verbindung der Formel II wird aus Gründen der Stabilität vorzugsweise als HSO₄⁻ Salz isoliert.

Die intramolekulare Cyclisierung bei der Verfahrenstufe ii) wird unter überraschend milden Reaktionsbedingungen erzielt und man erhält die erfindungsgemässen Verbindungen in hohen Ausbeuten und Reinheiten.

Ein anderer Gegenstand der Erfindung ist die Verbindung 4,8-Dibrom-1,5-diisothiocyanatonaphthalin der Formel III

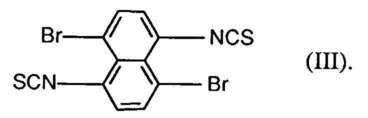

Die Verbindungen der Formel I können elektrolytisch oder chemisch durch Umsetzung mit einem Oxydationsmittel in Kationen übergeführt werden und bilden elektrisch leitende Charge-Transfer Komplexe mit verschiedenen Anionen.

Die erfindungsgemässen Verbindungen der Formel I bilden mit anorganischen Anionen Charge-Transfer Komplexe mit stöchiometrischer oder nicht-stöchiometrischer Zusammesetzung.

Ein weiterer Gegenstand der Erfindung sind Charge-Transfer Komplexe der Formel IV

ZₓA_{y} (IV),

worin Z das Radikalkation einer Verbindung der Formel I und A das Anion einer anorganischen Säure bedeuten, und 1 ≦ x/y ≦ 3. Die Stöichiometrie (Verhältnis Kation der Verbindungen der Formel I und Gegenanion) liegt bevorzugt zwischen 1:1 und 2:1.

Bei den anorganischen Säuren handelt es sich bevorzugt um einbasische Säuren, z.B. Mineralsäuren, Sauerstoffsäuren und Komplexsäuren.

Geeignete Anionen sind, z.B.
F⁻, Cl⁻, Br⁻, I⁻, CN⁻, OCN⁻, SCN⁻, SeCN⁻, N₃⁻, I₃⁻, I₂Br⁻, IBr₂⁻, BrICl⁻, Br₃⁻, ICl₂⁻, CuCl₂⁻, CuBr₂⁻, AgCl₂⁻, AgBr₂⁻, Agl₂⁻, Ag(CN)₂⁻, AuCl₂⁻, AuBr₂⁻, AuI₂⁻, Au(CN)₂⁻, NO₃⁻, C(CN)₃⁻, ClO₄⁻, BrO₄⁻, IO₄⁻, ReO₄⁻, FSO₃⁻, PO₂F₂⁻, BF₄⁻, InBr₄⁻, Inl₄⁻, TlBr₄⁻, TlI₄⁻, FeCl₄⁻, AuCl₄⁻, AuBr₄⁻, ICl₄⁻, SiF₅⁻, TeF₅⁻, PF₆⁻, AsF₆⁻, SbF₆⁻, SbCl₆⁻, NbF₆⁻ und TaF₆⁻.

Bevorzugte Anionen sind I₃⁻, IBr₂⁻, Br₃⁻, CuCl₂⁻ und PF₆⁻, besonders bevorzugt ist IBr₂⁻.

Die Herstellung von Charge-Transfer Komplexsalzen kann elektrolytisch oder chemisch durchgeführt werden. Bei der elektrolytischer Methode kann man wie folgt vorgehen: die Verbindung der Formel I wird in den Anodenraum einer Elektrolysezelle gefüllt, in der sich ein Leitelektrolyt mit einem Salz des anorganischen Anions, z.B. Tetraalkylammoniumsalzen wie Tetrabutylammonium-hexafluorophosphat, und ein Lösungsmittel, z.B. Dichlormethan, befindet. Man elektrolysiert bei Stromstärken von etwa 0,5 µA und nach einiger Zeit, z.B. 1 bis 2 Tagen, können die Kristalle von der Elektrode entfernt und gereinigt werden.

Bei der chemischen Methode kann man wie folgt vorgehen: eine gegebenenfalls heisse Lösung einer Verbindung der Formel I in einem Lösungsmittel, z.B. Toluol, wird mit einer Lösung eines Oxydationsmittels, z.B. Halogen, CuCl₂, FeCl₃, vermischt. Nach dem Abkühlen der Reaktionslösung werden die ausgefallenen Kristalle abfiltriert und gereinigt.

Die erfindungsgemässen Charge-Transfer Komplexe weisen ausgezeichnete elektrische Leitfähigkeiten auf und können als elektrische Leiter verwendet werden.

Ein weiterer Gegenstand der Erfindung ist die Verwendung der Charge-Transfer Komplexe der Formel IV als elektrische Leiter.

Die Charge-Transfer Komplexe der Formel IV können zum Beispiel zur Beschichtung von Oberflächen verwendet werden (antistatische Ausrüstung) oder sie können Kunststoffen als elektrisch leitende Füllstoffe einverleibt werden. Ferner ist es möglich, mono- oder multi-molekulare Schichten von Verbindungen der Formel I nach dem Langmuir-Blodgett Verfahren auf Substraten aufzubringen und diese Schichten zum Beispiel mit Halogenen zu dotieren. Solche Substrate mit dünnen elektrisch leitenden Schichten eignen sich als Basismaterial zur Herstellung von Sensoren.

Die nachfolgenden Beispiele erläutern die Erfindung näher.

### A. Herstellungsbeispiele

### Beispiel A1:

### a) Herstellung von 4,8-Dibrom-1,5-diaminonaphthalin

15 g (0,024 mol) 4,8-Dibrom-1,5-ditoluol-p-sulfonamidonaphthalin (J. S. Whitehurst, J. Chem. Soc., 221-226 (1951)) werden in 75 ml konzentrierter H₂SO₄ gelöst und die Lösung unter Lichtausschluss 24 h stehen gelassen. Danach wird auf 450 g Eis gegossen. Dabei ensteht eine violette Lösung aus der nach wenigen Minuten das Produkt als HSO₄-Salz ausfällt. Man erhält nach Abnutschen und Abpressen ein hellbeige-violettes Rohprodukt, das sofort weiter verarbeitet wird.

### b) Herstellung von 4,8-Dibrom-1,5-diisothiocyanatonaphthalin

Das obige Rohprodukt von 4,8-Dibrom-1,5-diaminonaphthalin in Form des HSO₄-Salzes wird in 150 ml H₂O suspendiert und mit 150 ml CH₂Cl₂ unterschichtet. Unter Rühren werden portionsweise 18 g Na₂CO₃ zugegeben. Zu dieser Emulsion wird bei Raumtemperatur (RT) eine Lösung von 6,3 g (0,054 Mol) Thiophosgen in 30 ml CH₂Cl₂ zugetropft. Nach 2 h Rühren bei RT ist die Reaktion beendet. Das Produkt wird mit je 600 ml Wasser und CH₂Cl₂ extrahiert. Die wässrige Phase wird nochmals mit 200 ml CH₂Cl₂ extrahiert; die organischen Phasen werden mit MgSO₄ getrocknet, filtriert und im Vakuum eingedampft. Der Rückstand (7,7 g) wird in 600 ml CH₂Cl₂ warm gelöst und die Lösung nach Abkühlen auf RT über 500 g Kieselgel und danach über Aktivkohle filtriert. Im Vakuum wird auf ein Volumen von 300 ml eingedampft und die ausgefallenen Kristalle werden abgenutscht. Ausbeute: 3,8 g (40%), Schmelzpunkt (Smp.): 202-204 °C. Das Kristallisat wird in 500 ml CH₂Cl₂ in der Wärme gelöst, auf 200 ml eingeengt und dann auskristallisiert. Nach Eiskühlung und Abnutschen werden 2,7 g der Titelverbindung mit einem Schmelzpunkt von 213.5-214 °C erhalten. MS (m/e): 400 (M+, 100%), IR (νₘₐₓ, KBr): 2095 cm⁻¹ (N=C=S). Aus der Mutterlauge sind noch weitere 0,35 g der Titelverbindung, Smp.: 207-209 °C (wegen der niedrigeren Qualität) isolierbar.

### c) Herstellung von 2',7'-Bis(methylthio)-naphtho[1,8-de:5,4-d'e']bis[1,3]thiazin

### (Methode "A")

In eine unter Argon gerührte Suspension von 200 mg (0,5 mMol) 4,8-Dibrom-1,5-diisothiocyanatonaphthalin in 4 ml absolutem Dimethylformamid werden bei 50 °C 100 mg (1,5 mMol) Natriummethanthiolat in Portionen zugegeben. Dabei löst sich das Edukt; das Reaktionsgemisch wird erst gelblich und dann orange, und das Produkt fällt aus der warmen Lösung aus. Es wird 15 min bei 50 °C nachgerührt, danach abgekühlt, abgenutscht und mit Wasser, Ethanol und Diethylether gewaschen. Das Rohprodukt wird in 80 ml heissem CH₂Cl₂ gelöst und über 800 g Kieselgel mit CH₂Cl₂/Hexan (1:1) chromatographiert. Die Hauptzone ergibt bei einer Ausbeute von 64,6% 108 mg orangefarbige Kristalle mit einem Smp. von 243,5-244 °C. MS (m/e): 334 (M⁺, 100%), IR (νₘₐₓ, KBr): 1558 cm⁻¹ (C=C).

### Beispiel A2:

### Herstellung von 2',7'-Bis(phenylthio)-naphtho[1,8-de:5,4-d'e']bis[1,3]thiazin

### (Methode "B")

In eine unter Argon gerührte Suspension von 200 mg (0,5 mMol) 4,8-Dibrom-1,5-diisocyanatonaphthalin und 130 mg (1,2 mMol) Triethylamin in 4 ml Dimethylformamid werden bei 50 °C unter Argon eine Lösung von 135 mg (1,2 mMol) Thiophenol in 4ml Dimethylformamid zugetropft. Dabei löst sich das Edukt, das Reaktionsgemisch wird gelblich, dann dunkelorange und das Produkt fällt aus der warmen Lösung aus. Nach 15 h bei 50 °C wird die Suspension abgekühlt, abgenutscht und mit Dimethylformamid, Wasser und Diethylether gewaschen. Das Rohprodukt wird aus 50 ml Toluol umkristallisiert, wobei 180 mg orangefarbige Kristalle, Smp.: 299,4 °C, erhalten werden. MS (m/e): 458 (M⁺, 100%), IR (νₘₐₓ, KBr): 1558 cm⁻¹ (C=C); Ausbeute 87,3%.

### Beispiel A3:

### Herstellung von 2'-n-Butylthio-4-brom-5-isothiocyanato-naphtho[1,8-de][1,3]thiazin

Zu einer Suspension von 400 mg (1 mMol) 4,8-Dibrom-1,5-diisothiocyanatonaphthalin und 130 mg (1,2 mMol) Triethylamin in 15 ml absolutem Tetrahydrofuran werden bei RT 110 mg (1,2 mMol) n-Butylmercaptan (97%ig) zugegeben und bis zum Rückfluss erwärmt. Während des Aufheizens entsteht zunächst eine homogene Reaktionsmischung. Nach 5 min fällt aus der gelborangen Lösung (C₂H₅)₃N HBr aus. Es wird 4 h unter Rückfluss erhitzt. Danach wird im Vakuum bis zur Trockne eingeengt, in Benzol/Hexan (1:2) aufgenommen und mit diesem Gemisch über 50 g Kieselgel chromatographiert. Die zitronengelbe Hauptzone enthält die Titelverbindung mit 213 mg (Ausbeute 51%) zitronengelber Kristalle, Smp.: 93,5-94 °C, MS (m/e): 410 (M⁺, 100%), IR (νₘₐₓ, KBr): 2110 (N=C=S), 1550 (C=C) cm⁻¹.

### Beispiele A4-A7:

Die nachfolgenden Verbindungen werden analog zu Beispiel A3 hergestellt und können zur Herstellung unsymmetrischer Verbindungen der Formel I verwendet werden.

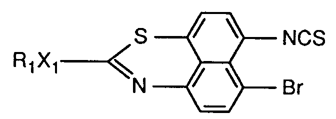

| **X₁** | **R₁** | **Smp.(°C)** | **Methode** |
|---|---|---|---|
| S | -(CH₂)₃CH₃ | 93,5-94 | B |
| S | -CH₃ | 172,5 | A |
| O | -CH₃ | 231-232 | A |
| S | -(CH₂)₁₁CH₃ | 84-84,5 | B |

### Beispiel A8:

### Herstellung von 2'-n-Butylthio-7'-methylthio-naphtho[1,8-de:5,4-d'e']bis[1,3]thiazin

Zu einer Lösung von 102 mg (0,25 mMol) der Titelverbindung gemäss Beispiel A3 in 3 ml Dimethylformamid werden bei 50 °C unter Argon 23 mg (0,33 mMol) CH₃SNa zu-gegeben. Die Lösung wird sofort rot. Nach 2 Std. bei 60 °C wird die Suspension abgekühlt, mit 10 ml Wasser verdünnt und mit CH₂Cl₂ extrahiert. Die Extraktionslösung wird mit Wasser gewaschen, über MgSO₄ getrocknet und im Vakuum eingedampft. Der Rückstand wird über 75 g Kieselgel chromatographiert (Benzol/Hexan, 1:2). Die zweite orangerote Zone enthält die Titelverbindung als orange Kristalle. Ausbeute 31 mg (33%), Smp.: 121,5-122 °C, MS (m/e): 376 (M⁺, 100%), IR (ν_{max,} KBr): 1556 cm⁻¹ (C=C).

### Beispiel A9-A20:

Die nachfolgenden Verbindungen werden analog zu Beispiel A8 hergestellt.

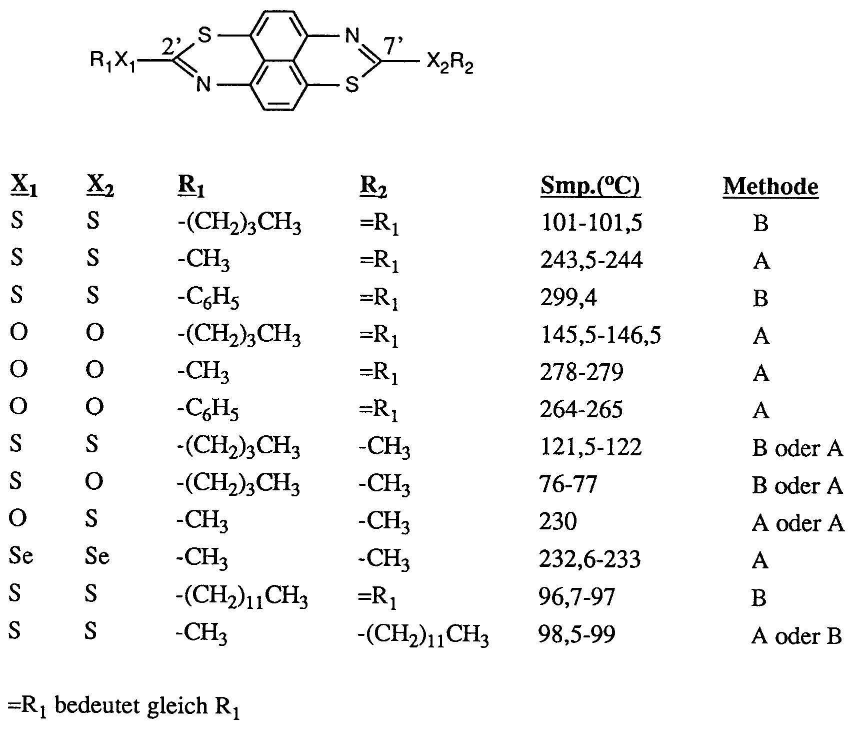

### B. Herstellung von Charge-Transfer Komplexsalzen

### Herstellung von [2',7'-Bis(methylthio)-naphtho[1,8-de:5,4-d'e']bis[1,3]thiazino]₂hexafluorophosphat

5 mg (0,015 mMol) 2',7'-Bis(methylthio)-naphtho[1,8-de:5,4-d'e']bis[1,3]thiazin werden in den Anodenraum einer Elektrolysezelle von 6 ml Volumen eingefüllt. Als Leitelektrolyt werden 15 mg (0,04 mMol) Tetrabutylammonium-PF6 verwendet und ebenfalls unter Argon in die Zelle eingefüllt. Als Lösungsmittel werden 3 ml Dichlormethan verwendet. Dann wird ein Strom von 0,5 µA eingestellt (Pt-Draht-Elektroden 1 x 10 mm). Nach zwei Tagen erhält man dicke, schwarze Nadeln der Titelverbindung. Die elektrische Leitfähigkeit dieses Komplexes als Einzelkristall beträgt σ_{RT}= 25 S/cm (gemessen nach der Vierpunktmethode). Die Stöchiometrie wird durch Röntgenstrukturanalyse bestätigt.

### Beispiel B2:

### Herstellung von [2',7'-Bis(methylthio)-naphtho[1,8-de:5,4-d'e']bis[1,3]thiazino](I₃)_{0,46}

Zu einer Lösung von 33,4 mg (0,1 mMol) 2',7'-Bis(methylthio)-naphtho[1,8-de:5,4-d'e']bis[1,3]thiazin in 3 ml heissem Toluol wird eine Lösung von 19 mg (0,075 mMol) Iod in 1 ml Toluol gegeben. Nach Abkühlen der Lösung, werden die erhaltenen goldenen Kris-talle abfiltriert, mit wenig Toluol gewaschen und an der Luft getrocknet. Ausbeute 48 mg (90%), Smp.: 205 °C. Die Leitfähigkeit eines Einzelkristalls beträgt 150 S/cm (gemessen nach der Vierpunktmethode). Die Zusammensetzung wird in einer Elementaranalyse ermittelt.

## Patentansprüche

1. Verbindungen der Formel I 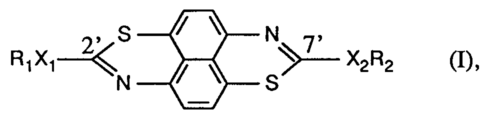 worin
X₁ und X₂ unabhängig voneinander O, S oder Se bedeuten,
und R₁ und R₂ unabhängig voneinander einen unsubstituierten oder mit NH2, C₁-C₆-Alkyl, C₁-C₆-Alkoxy, Phenyl oder C₃-C₈-Cycloalkyl substituierten einwertigen Rest eines aliphatischen oder aromatischen Kohlenwasserstoffs mit 1 bis 20 C-Atomen darstellen.

2. Verbindungen der Formel I gemäss Anspruch 1, dadurch gekennzeichnet, dass X₁ und X₂ gleich sind.

3. Verbindungen der Formel I gemäss Anspruch 2, dadurch gekennzeichnet, dass X₁ und X₂ S bedeuten.

4. Verbindungen der Formel I gemäss Anspruch 2, dadurch gekennzeichnet, dass X₁ und X₂ O bedeuten.

5. Verbindungen der Formel I gemäss Anspruch 1, dadurch gekennzeichnet, dass es sich bei dem Kohlenwasserstoffrest für R₁ und/oder R₂ um C₁-C₁₈-Alkyl, C₃-C₈-Cycloalkyl, C₇-C₁₂-Aralkyl oder C₆-C₁₈-Aryl handelt.

6. Verbindungen der Formel I gemäss Anspruch 5, dadurch gekennzeichnet, dass es sich bei den aliphatischen Kohlenwasserstoffresten um C₁-C₁₂-Alkyl, C₄-C₆-Cycloalkyl oder C₇-C₁₂-Aralkyl, und bei den aromatischen Kohlenwasserstoffresten um C₆-C₁₂-Aryl handelt.

7. Verbindungen der Formel I gemäss Anspruch 1, dadurch gekennzeichnet, dass R₁ und R₂ unsubstituiertes oder wie in Anspruch 1 substituiertes C₁-C₁₈-Alkyl, C₅- oder C₆-Cycloalkyl, Phenyl oder Benzyl bedeuten.

8. Verbindungen der Formel I gemäss Anspruch 1, dadurch gekennzeichnet, dass R₁ und R₂ C₁-C₁₂-Alkyl, Phenyl oder Benzyl bedeuten.

9. Eine Verbindung der Formel I gemäss Anspruch 1, dadurch gekennzeichnet, dass es sich um 2',7'-Bis(methylthio)-naphtho[1,8-de:5,4-d'e']bis[1,3]thiazin handelt.

10. Eine Verbindung der Formel I gemäss Anspruch 1, dadurch gekennzeichnet, dass es sich um 2',7'-Bis(methoxy)-naphtho[1,8-de:5,4-d'e']bis[1,3]thiazin handelt.

11. Eine Verbindung der Formel I gemäss Anspruch 1, dadurch gekennzeichnet, dass es sich um 2'-Methylthio-7'-ethoxy-naphtho[1,8-de:5,4-d'e']bis[1,3]thiazin handelt.

12. Eine Verbindung der Formel I gemäss Anspruch 1, dadurch gekennzeichnet, dass es sich um 2'-n-Butylthio-7'-methylthio-naphtho[1,8-de:5,4-d'e']bis[1,3]thiazin handelt.

13. Verfahren zur Herstellung von Verbindungen der Formel I gemäss Anspruch 1, dadurch gekennzeichnet, dass man
i) 4,8-Dibrom-1,5-diaminonaphthalin der Formel II, gegebenenfalls als Salz, 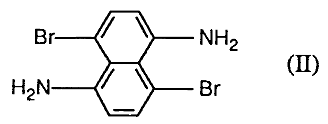 in Gegenwart eines inerten Lösungsmittels und Na₂CO₃ mit Thiophosgen zu 4,8-Dibrom-1,5-diisothiocyanatonaphthalin der Formel III 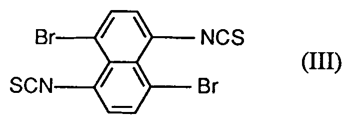 umsetzt,
ii) das 4,8-Dibrom-1,5-diisothiocyanatonaphthalin der Formel III in Gegenwart eines Lösungsmittels entweder durch Umsetzung mit 2 Äquivalenten eines einwertigen Metall- oder Ammoniumsalzes R₁X₁⁻ M⁺ oder eines Erdalkalimetallsalzes (R₁X₁⁻)₂E²⁺ in eine Verbindung der Formel Ia 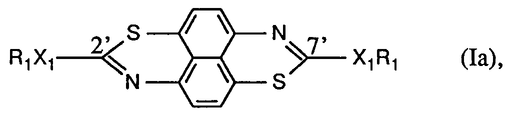 umwandelt, worin M⁺ ein einwertiges Metallkation oder ein Ammoniumkation R₃R₄R₅R₆N⁺ darstellt und R₃, R₄, R₅ und R₆ unabhängig voneinander H oder C₁-C₄-Alkyl und E²⁺ ein Erdalkalimetallkation bedeuten,
oder stufenweise erst durch Umsetzung mit 1 Äquivalent eines Salzes eines einwertigen Metall- oder Ammoniumsalzes R₁X₁⁻M⁺ oder eines Erdalkalimetallsalzes (R₁X₁⁻)₂E²⁺ und dann mit 1 Äquivalent eines anderen einwertigen Metall- oder Ammoniumsalzes R₂X₂⁻M⁺ oder eines anderen Erdalkalimetallsalzes (R₂X₂⁻)₂E²⁺, in eine Verbindung der Formel I 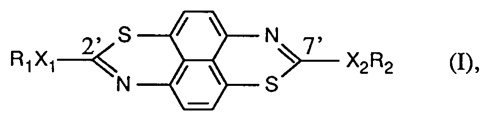 umwandelt, worin R₁, R₂, X₁ und X₂ die zuvor angegebenen Bedeutungen haben.

14. Die Verbindung 4,8-Dibrom-1,5-diisothiocyanatonaphthalin der Formel III

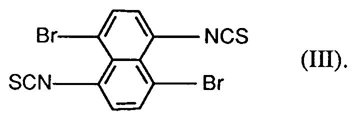

15. Charge-Transfer Komplexe der Formel IV
ZₓA_{y} (IV),
worin Z das Radikalkation einer Verbindung der Formel I gemäss Anspruch 1 und A das Anion einer anorganischen Säure bedeuten, und 1 ≦ x/y ≦ 3.

16. Charge-Transfer Komplexe der Formel IV gemäss Anspruch 15, dadurch gekennzeichnet, dass es sich bei dem Anion A um F⁻, Cl⁻, Br⁻, I⁻, CN⁻, OCN⁻, SCN⁻, SeCN⁻, N₃⁻, I₃⁻, I₂Br⁻, IBr₂⁻, BrICl⁻, Br₃⁻, ICl₂⁻, CuCl₂⁻, CuBr₂⁻, AgCl₂⁻, AgBr₂⁻, AgI₂⁻, Ag(CN)₂⁻, AuCl₂⁻, AuBr₂⁻, AuI₂⁻, Au(CN)₂⁻, NO₃⁻, C(CN)₃⁻, ClO₄⁻, BrO₄⁻, IO₄⁻, ReO₄⁻, FSO₃⁻, PO₂F₂⁻, BF₄⁻, InBr₄⁻, InI₄⁻, TlBr₄⁻, TlI₄⁻, FeCl₄⁻, AuCl₄⁻, AuBr₄⁻, ICl₄⁻, SiF₅⁻, TeF₅⁻, PF₆⁻, AsF₆⁻, SbF₆⁻, SbCl₆⁻, NbF₆⁻ oder TaF₆⁻ handelt.

17. Ein Charge-Transfer Komplex gemäss Anspruch 15, dadurch gekennzeichnet, dass es sich um [2',7'-Bis(methylthio)-naphtho[1,8-de:5,4-d'e']bis[1,3] thiazino]₂[hexafluorophosphat handelt.

18. Ein Charge-Transfer Komplex gemäss Anspruch 15, dadurch gekennzeichnet, dass es sich um [2',7'-Bis(methylthio)-naphtho[1,8-de:5,4-d'e']bis[1,3]thiazino] (I₃)_{0,46} handelt.

19. Verwendung der Charge-Transfer Komplexe der Formel IV gemäss Anspruch 15 als elektrische Leiter.
